# EUROPEAN PATENT APPLICATION

(11) **EP 3 682 869 A1**
(43) Date of publication of application: **22.07.2020**
(21) Application number: 20160560.7
(22) Date of filing: 19.03.2009
(51) Int. Cl.: A61K 9/14

(54) **PROCESS FOR OBTAINING POWDER COMPOSITIONS OF ORLISTAT**

(30) Priority: 20.03.2008 PL 38475608
(62) Divisional of application: 09721857.2
(71) Applicant: Zaklady Farmaceutyczne Polpharma S.A., 83-200 Starogard Gdanski (PL)
(72) Inventor: MAZGALSKI, Jaroslaw, 83-010 Straszyn (PL); SZEMECKA-GOLABEK, Anna, 83-212 Dabrowka (PL); KAMINSKA, Anna, 83-200 Starogard Gdanski (PL)
(74) Representative: Isarpatent

(57) **Abstract**

The invention relates to a novel process for the manufacture of stable, meeting relevant quality requirements pharmaceutical compositions in the form of encapsulated powder comprising Orlistat and pharmaceutically acceptable additives. The invention further relates to pharmaceutical compositions of Orlistat in the form of encapsulated powder obtained in accordance with the process of the present invention.

## Description

The invention relates to a novel process for the manufacture of stable, meeting relevant quality requirements pharmaceutical compositions in the form of encapsulated powder comprising Orlistat and pharmaceutically acceptable additives. The invention further relates to pharmaceutical compositions of Orlistat in the form of encapsulated powder obtained in accordance with the process of the present invention.

Orlistat, known as tetrahydrolipstatin or THL, is an inhibitor of pancreatic lipase. Orlistat acts within the GI tract to inhibit the enzymes which hydrolyze dietary fat to glycerol and fatty acids and hence prevents absorption of lipids into the bloodstream. For this reason, Orlistat is indicated for the treatment of obesity in patients with body mass index (BMI) of greater or equal to 30 kg/m².

Due to distinct physicochemical properties, namely waxy character, melting point at 44 °C and low chemical stability, Orlistat is considered in the state of art as a technologically demanding and difficult to handle active ingredient. Known in the state of art methods for obtaining pharmaceutical compositions comprising Orlistat employ complicated and expensive technologies, for instance, extrusion and spheronisation disclosed in International patent application WO 98/34607 or expansion of a solution or homogeneous dispersion comprising Orlistat under reduced pressure without boiling disclosed in International patent application WO 02/00201. Similarly, known in the state of art pharmaceutical compositions comprising Orlistat teach to employ in the formulations of the active ingredient rarely used in practice, atypical, expensive and often unstable pharmaceutical additives in order to obtain a product meeting relevant quality requirements. Such atypical pharmaceutical additives are, for example, fatty acid esters of polyols disclosed in International patent application WO 01/19378, sucrose fatty acid esters disclosed in International patent application WO 02/098412, fatty acids or fatty acid salts or mixtures of fatty acids and fatty acid salts disclosed in International patent application WO 02/098413, bile acid sequestrants disclosed in International patent application WO 02/09815, poorly digestible, poorly fermentable, hydrophilic and/or hydrocolloidal food grade thickeners disclosed in International patent application WO 00/09122 and complex surfactants disclosed in International patent application WO 01/19340.

The above mentioned publications clearly indicate the need of employing in the manufacture of pharmaceutical compositions of Orlistat complicated technologies and/or , atypical, often rendering technological difficulties and expensive pharmaceutical additives. Also Roche's reference product, Xenical®, manufactured using extrusion and spheronisation method, which is regarded as a relatively complicated, time-consuming, demanding supervision of experienced technologists and expensive technology, is a strong confirmation of the discussed presumption.

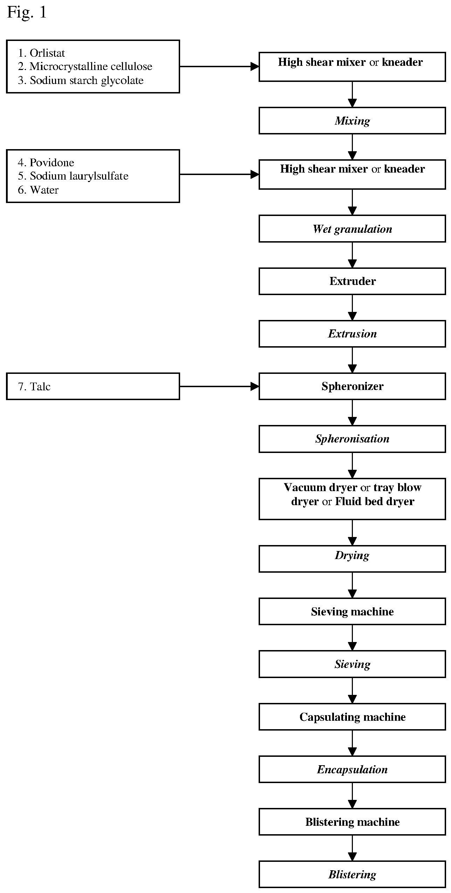

The extrusion and spheronisation technology employed by Roche in the manufacture of Xenical® comprises numerous critical steps which have direct influence on chemical purity and final quality of the dosage form. These critical steps are, for example, wet granulation stage (due to the fact that Orlistat readily undergoes hydrolysis) and extrusion stage, which is accompanied by temperature rise (since Orlistat readily oxidizes and has melting point at temperature as low as 44 °C). A scheme of the manufacturing process of Roche's Xenical® by extrusion and spheronisation method is presented in Fig. 1.

In addition, the formulation of Roche's Xenical® comprises micronized Orlistat, i.e. having greater total surface when compared to unmicronized active ingredient. As a result of this feature, the active ingredient present in the formulation of Xenical® is much more prone to the above indicated undesired phenomena. Therefore, to reduce their influence on the manufacturing process of Xenical®, the process requires performing in non-standard conditions, for instance some stages of the process are carried out under inert atmosphere or under vacuum. For this reason, the discussed manufacturing process is regarded as strongly complicated and expensive.

Surprisingly, it was found that pharmaceutical capsule compositions of Orlistat can be manufactured using relatively simple, inexpensive and fast process comprising blending Orlistat and pharmaceutically acceptable additives and encapsulating thus obtained pharmaceutical powder composition without the need of employing in the composition atypical, often rendering technological difficulties and expensive pharmaceutical additives. It was also found that the process according to the present invention provides pharmaceutical powder compositions comprising Orlistat of higher chemical stability than Xenical®. The above feature is an effect of better protection of the active ingredient Orlistat against chemical degradation in the composition obtained by the process according to the invention.

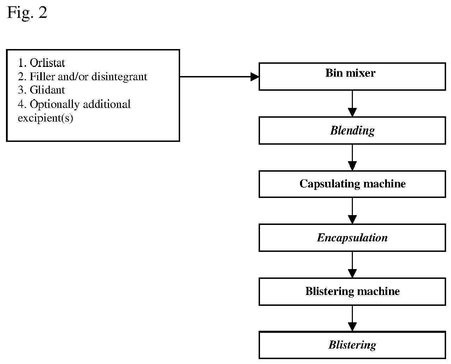

The invention relates to a process for the manufacture of pharmaceutical compositions in the form of encapsulated powder comprising Orlistat and pharmaceutically acceptable additives which comprises two steps. In the first step of the process according to the invention Orlistat is blended with pharmaceutically acceptable additives selected from the group comprising a glidant and a disintegrant or a filler or, preferably, a mixture of a disintegrant and a filler and, optionally, one or more additional pharmaceutically acceptable additives to obtain a homogeneous powder. In the second step of the process the homogeneous powder obtained in the first step is encapsulated. A scheme of the manufacturing process of pharmaceutical compositions comprising Orlistat according to the invention is presented in Fig. 2.

The process for the manufacture of pharmaceutical compositions comprising Orlistat according to the invention does not require employing atypical, often rendering technological difficulties and expensive pharmaceutical additives, the use of which is implied in the prior art. The process according to the invention allows for employing in the manufacture of pharmaceutical composition comprising Orlistat typical, widely used in practice pharmaceutically acceptable additives , which greatly simplifies the manufacturing process and reduces costs.

In a preferred embodiment of the invention, the pharmaceutical powder composition manufactured according to the process of the invention comprises Orlistat in an amount of 30 to 70% by weight of the powder composition. In another preferred embodiment of the invention, the pharmaceutical powder composition manufactured according to the process of the invention comprises Orlistat in an amount of 120 mg per dose. In yet another preferred embodiment of the invention, the pharmaceutical powder composition manufactured according to the process of the invention comprises Orlistat in an amount of 60 mg per dose.

The process for the manufacture of pharmaceutical powder compositions comprising Orlistat according to the invention does not encompass employing in the process micronized active ingredient. The above is clearly an advantage of the process according to the invention due to significant technological difficulties with micronizing Orlistat resulting from its waxy character. In addition, the process of the invention does not require employing in the powder compositions Orlistat active ingredient of a specific and narrow range of particle size, in particular, the process does not require eliminating from the compositions largest particles present in commercially available Orlistat, i.e. particles of size in the range of 200 to 300 µm. In accordance to the above, the process of the invention involves employing in the powder composition Orlistat active ingredient having a D₉₀ particle size of more than 30 µm.

In a preferred embodiment of the invention, the pharmaceutical powder composition manufactured according to the process of the invention comprises a glidant in an amount of 1 to 10% by weight of the powder composition. In another preferred embodiment of the invention, the pharmaceutical powder composition comprises a glidant selected from the group consisting of anhydrous silica dioxide, talc, magnesium stearate , calcium stearate, stearic acid, sodium stearyl fumarate, glyceryl behenate, glyceryl palmitostearate, sodium laurylsulfate and mixtures thereof.

The process for the manufacture of pharmaceutical powder compositions comprising Orlistat according to the invention involves employing in the composition a disintegrant or a filler or a mixture of a disintegrant and a filler. In a preferred embodiment of the invention, the powder composition comprises a mixture of a disintegrant and a filler.

In a preferred embodiment of the invention, the pharmaceutical powder composition manufactured according to the process of the invention comprises a disintegrant in an amount of 3 to 30% by weight of the powder composition. In another preferred embodiment of the invention, the pharmaceutical powder composition comprises a disintegrant selected from the group consisting of sodium starch glycolate, croscarmellose, crospovidone and mixtures thereof.

In a preferred embodiment of the invention, the pharmaceutical powder composition manufactured according to the process of the invention comprises a filler in an amount of 10 to 80% by weight of the powder composition. In another preferred embodiment of the invention, the pharmaceutical powder composition comprises a filler selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof. In yet another preferred embodiment of the invention, the pharmaceutical powder composition comprises a filler selected from the group consisting of low moisture content microcrystalline cellulose, low moisture content maize starch, low moisture content potato starch and mixtures thereof.

Low moisture content fillers mentioned above are characterized by reduced free water content when compared to standard fillers of the same type. For instance, low moisture content microcrystalline cellulose having free water content of less than 1,5% is available from JRS Pharma under the trade name Vivapur PH 112, low moisture content corn starch having free water content of 7,5% is available from Roquette under the trade name Corn starch 400L NF, low moisture content potato starch having free water content of 8,0% is available from Roquette under the trade name Potato starch 8,0%.

In a preferred embodiment of the invention, the pharmaceutical powder composition manufactured according to the process of the invention comprises one or more additional pharmaceutically acceptable additives. In another preferred embodiment of the invention, the pharmaceutical powder composition comprises a surfactant as the additional pharmaceutically acceptable additive present in the powder composition in an amount of 0,1 to 5% by weight. In yet another preferred embodiment of the invention, the surfactant employed as the additional pharmaceutically acceptable additive is sodium lauryl sulfate.

The following examples illustrate various aspects of the present invention. They are not to be construed to limit the claims in any manner whatsoever.

### Example 1

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per caps.** |
|---|---|---|---|
| Orlistat | 1,800 | 120,00 | 52,17 |
| Vivapur PH 112 | 0,856 | 57,10 | 24,83 |
| Sodium starch glycolate type A | 0,621 | 41,40 | 18,00 |
| Anhydrous silica dioxide | 0,138 | 9,20 | 4,00 |
| Sodium lauryl sulfate | 0,035 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 2

Manufacturing process of encapsulated powder composition comprising Orlistat for a batch size of 3,45 kg (30 000 capsules, 115 mg each) comprising the following ingredients.

| **Ingredient** | **Amount [kg]** | **Amount [mg/caps.]** | **% per capsule** |
|---|---|---|---|
| Orlistat | 1,800 | 60,00 | 52,17 |
| Mannitol | 0,930 | 31,00 | 26,96 |
| Crospovidone | 0,345 | 11,50 | 10,00 |
| Corn starch 400L NF | 0,2025 | 6,75 | 5,87 |
| Anhydrous silica dioxide | 0,138 | 4,60 | 4,00 |
| Sodium lauryl sulfate | 0,0345 | 1,15 | 1,00 |
| **TOTAL** | **3,450** | **115,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 3 using rotary capsulating machine. Hard gelatin capsule composition comprising 60 mg of Orlistat in 115 mg of powder was obtained.

### Example 3

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per capsule** |
|---|---|---|---|
| Orlistat | 1,800 | 120,00 | 52,17 |
| Dibasic calcium phosphate dihydrate | 0,7385 | 49,23 | 21,41 |
| Sodium starch glycolate type A | 0,739 | 49,27 | 21,42 |
| Anhydrous silica dioxide | 0,138 | 9,20 | 4,00 |
| Sodium lauryl sulfate | 0,0345 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 4

Manufacturing process of encapsulated powder composition comprising Orlistat for a batch size of 3,2 kg (20 000 capsules, 160 mg each) comprising the following ingredients.

| **Ingredient** | **Amount [kg]** | **Amount [mg/caps.]** | **% per capsule** |
|---|---|---|---|
| Orlistat | 2,400 | 120,00 | 75,00 |
| Lactose anhydrous | 0,512 | 25,60 | 16,00 |
| Sodium starch glycolate type A | 0,160 | 8,00 | 5,00 |
| Anhydrous silica dioxide | 0,064 | 3,20 | 2,00 |
| Sodium lauryl sulfate | 0,064 | 3,20 | 2,00 |
| **TOTAL** | **3,200** | **160,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 2 using rotary capsulating machine. Hard gelatin capsule composition comprising 120 mg of Orlistat in 160 mg of powder was obtained.

### Example 5

Manufacturing process of encapsulated powder composition comprising Orlistat for a batch size of 3,45 kg (15 000 capsules, 230 mg each) comprising the following ingredients.

| **Ingredient** | **Amount [kg]** | **Amount [mg/caps.]** | **% per capsule** |
|---|---|---|---|
| Orlistat | 1,800 | 120,00 | 52,17 |
| Corn starch 400L NF | 0,856 | 57,10 | 24,83 |
| Sodium starch glycolate type A | 0,621 | 41,40 | 18,00 |
| Anhydrous silica dioxide | 0,138 | 9,20 | 4,00 |
| Sodium lauryl sulfate | 0,035 | 2,30 | 1,00 |
| **TOTAL** | **3,450** | **230,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 1 using rotary capsulating machine. Hard gelatin capsule composition comprising 120 mg of Orlistat in 230 mg of powder was obtained.

### Example 6

Manufacturing process of encapsulated powder composition comprising Orlistat for a batch size of 3,45 kg (30 000 capsules, 115 mg each) comprising the following ingredients.

| **Ingredient** | **Amount [kg]** | **Amount [mg/caps.]** | **% per capsule** |
|---|---|---|---|
| Orlistat | 1,800 | 60,00 | 52,17 |
| Corn starch 400L NF | 0,856 | 28,55 | 24,83 |
| Sodium starch glycolate type A | 0,621 | 20,70 | 18,00 |
| Anhydrous silica dioxide | 0,138 | 4,60 | 4,00 |
| Sodium lauryl sulfate | 0,035 | 1,15 | 1,00 |
| **TOTAL** | **3,450** | **115,00** | **100,00** |

The above ingredients in given amounts were passed through a sieve mesh size 1,0 mm and blended in a 20-litre bin mixer for 30 minutes at the speed of 12 rpm until a homogeneous powder mixture was obtained. The powder composition was next encapsulated into capsules size 3 using rotary capsulating machine. Hard gelatin capsule composition comprising 60 mg of Orlistat in 115 mg of powder was obtained.

The invention relates also to pharmaceutical compositions in the form of encapsulated powder comprising Orlistat obtained in accordance with the process of the present invention.

Pharmaceutical compositions in the form of encapsulated powder comprising Orlistat obtained in accordance with the process of the present invention effectively protect Orlistat, which is regarded as a highly chemically unstable active ingredient, against chemical degradation. The above is well demonstrated by comparing results of the stability tests of the composition prepared in accordance with the process of the present invention presented in example 1 and the reference product, Roche's Xenical®.

Testing of chemical stability of pharmaceutical compositions comprising Orlistat is carried out by measuring the increase of content of the main product of degradation of Orlistat, i.e. 5-[(N-formylleucyl)-oxy]-2-hexyl-3-hydroxyhexadecanic acid, also known as delactone, in time. The results of stability tests performed after storing both tested products in the conditions of, respectively, the temperature at 40 °C and 75% relative humidity and the temperature at 30 °C and 65% relative humidity are given below.

| **Delactone content** | **Storing conditions: 40 °C and 75% rh** | | | |
|---|---|---|---|---|
| | T₀ | 1 month | 2 months | 3 months |
| **Composition of example 1 in PVDC packaging** | BRT | 0,33 | 0,97 | 3,26 |
| **Xenical®** | 0,39 | 0,89 | 3,88 | 7,22 |

| | | | | |
|---|---|---|---|---|
| BRT - below reporting threshold | | | | |

| **Delactone content** | **Storing conditions: 30 °C and 65% rh** | | | | |
|---|---|---|---|---|---|
| | T₀ | 1 month | 2 months | 3 months | 6 months |
| **Composition of example 1 in PVDC packaging** | BRT | BRT | BRT | BRT | 0,11 |
| **Xenical®** | 0,39 | 0,44 | 0,49 | 0,54 | 0,65 |

| | | | | | |
|---|---|---|---|---|---|
| BRT - below reporting threshold | | | | | |

The above presented stability data clearly demonstrate that pharmaceutical compositions in the form of encapsulated powder comprising Orlistat and typical, widely used pharmaceutically acceptable additives manufactured in accordance with the relatively simple and inexpensive process of the present invention have significantly higher chemical stability than the reference product Xenical®, which is manufactured using complicated and expensive extrusion and spheronisation method.

The invention relates also to the use of Orlistat having a D₉₀ particle size of more than 30 µm in the manufacture of pharmaceutical powder compositions by means of blending Orlistat and pharmaceutically acceptable additives selected from the group comprising a glidant and a disintegrant or a filler or, preferably, a mixture of a disintegrant and a filler as defined above.

## Claims

1. A process for the manufacture of pharmaceutical compositions in the form of encapsulated powder comprising Orlistat and pharmaceutically acceptable additives selected from the group comprising a glidant and a disintegrant or a filler or, preferably, a mixture of a disintegrant and a filler, **characterized in that** the process comprises the steps of obtaining pharmaceutical powder by blending Orlistat and pharmaceutically acceptable additives and encapsulating thus obtained powder.

2. Process according to claim 1, wherein Orlistat is present in the powder composition in an amount of 30 to 70% by weight, preferably in an amount of 1 to 10% by weight.

3. Process according to claim 2, wherein the glidant is selected from the group consisting of anhydrous silica dioxide, talc, magnesium stearate , calcium stearate, stearic acid, sodium stearyl fumarate, glyceryl behenate, glyceryl palmitostearate, sodium laurylsulfate and mixtures thereof.

4. Process according to claim 1, wherein the disintegrant is present in the powder composition in an amount of 3 to 30% by weight, and preferably is selected from the group consisting of sodium starch glycolate, croscarmellose, crospovidone and mixtures thereof.

5. Process according to claim 1, wherein the filler is present in the powder composition in an amount of 10 to 80% by weight, and preferably is selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof.

6. Process according to claim 5, wherein the filler is preferably selected from the group consisting of low moisture content microcrystalline cellulose, low moisture content maize starch, low moisture content potato starch and mixtures thereof.

7. Process according to claim 1, wherein the powder composition comprises one or more additional pharmaceutically acceptable additives.

8. Process according to claim 7, wherein the additional pharmaceutically acceptable additive is a surfactant present in the powder composition in an amount of 0,1 to 5% by weight, wherein the surfactant preferably is sodium lauryl sulfate.

9. Pharmaceutical composition in the form of encapsulated powder comprising Orlistat manufactured according to a process as defined in any of the claims 1 to 8.

10. The use of Orlistat having a D₉₀ particle size of more than 30 µm in the manufacture of pharmaceutical powder compositions by means of blending Orlistat and pharmaceutically acceptable additives selected from the group comprising a glidant and a disintegrant or a filler or, preferably, a mixture of a disintegrant and a filler.

11. The use of Orlistat according to claim 10, wherein Orlistat is present in the powder composition in an amount of 30 to 70% by weight.

12. The use of Orlistat according to claim 10, wherein the glidant is present in the powder composition in an amount of 1 to 10% by weight and is selected from the group consisting of anhydrous silica dioxide, talc, magnesium stearate , calcium stearate, stearic acid, sodium stearyl fumarate, glyceryl behenate, glyceryl palmitostearate, sodium laurylsulfate and mixtures thereof.

13. The use of Orlistat according to claim 10, wherein the disintegrant is present in the powder composition in an amount of 3 to 30% by weight and is selected from the group consisting of sodium starch glycolate, croscarmellose, crospovidone and mixtures thereof.

14. The use of Orlistat according to claim 10, wherein the filler is present in the powder composition in an amount of 10 to 80% by weight and is selected from the group consisting of microcrystalline cellulose, silicified microcrystalline cellulose, powdered cellulose, maize starch, modified maize starch, potato starch, lactose monohydrate, lactose anhydrous, mannitol, dibasic calcium phosphate dihydrate, anhydrous dibasic calcium phosphate, magnesium carbonate, erythritol, trehalose and mixtures thereof, preferably low moisture content microcrystalline cellulose, low moisture content maize starch, low moisture content potato starch or a mixture thereof.

15. The use of Orlistat according to claim 10, wherein the powder composition comprises one or more additional pharmaceutically acceptable additives, preferably a surfactant present in the composition in an amount of 0,1 to 5% by weight, preferably sodium lauryl sulfate.
